# EUROPEAN PATENT SPECIFICATION

(11) **EP 1 575 458 B1**
(45) Date of publication and mention of the grant of the patent: **25.02.2009**
(21) Application number: 03790406.7
(22) Date of filing: 08.12.2003
(51) Int. Cl.: A61F 2/44, A61F 2/46, A61M 25/10

(54) **APPARATUS FOR INTERVERTEBAL DISC EXPANSION**
GERÄT ZUR BANDSCHEIBENEXPANSION
APPAREIL D'EXTENSION DE DISQUE INTERVERTEBRAL

(30) Priority: 07.12.2002 US 314396
(43) Date of publication of application: 21.09.2005
(73) Proprietor: Warsaw Orthopedic, Inc., Warsaw, IN 46581 (US); Trieu, Hai H., Cordova, MN 38018 (US)
(72) Inventor: TRIEU, Hai, H., Cordova, MN 38018 (US)
(74) Representative: O'Connell, Maura
(86) International application number: PCT/US2003/038957
(87) International publication number: WO 2004/052248

(56) References cited:
- WO-A-02/17825
- DE-A- 19 959 975
- US-A- 6 099 514
- US-A1- 2002 045 942
- US-A1- 2002 177 866
- US-B1- 6 264 659
- US-B1- 6 436 119

## Description

Degenerated disc disease (DDD) leads to disc dehydration (black disc), gradual collapse, and ultimately leg and/or back pain. Interbody fusion is the current standard of care for DDD. It is desirable that this end-stage treatment be delayed as long as possible by early intervention with less invasive approaches. Disc augmentation by injection of a biomaterial into the disc space has been proposed previously as an early minimally invasive treatment for a degenerated disc. Depending on the level of dehydration and collapse, injection of a biomaterial into the disc space of an intact disc (uncompromised annulus with no significant tears and original nucleus pulposus still in place) may require a high injection pressure and the injectable volume of biomaterial may be limited. High injection pressure increases the overall risk of the procedure including leakage, disc rupture, etc. Limited injectable volume reduces the effectiveness of the treatment and may require multiple treatments to achieve desirable results.

In known methods for intervertebral disc expansion, a cut is made in the disc annulus and disc tissue is removed to provide a passage for the insertion of an expansion device, an expansion material, or both. Also, the nucleus pulposus is removed and replaced by the expansion material and/or expansion device. Furthermore, degeneration of the disc is accelerated when an opening is cut into the disc annulus and tissue is removed.

WO 02/17825 discloses a balloon catheter expansion device according to the preamble of claim 1.

What is needed is a device and method for accessing the nucleus pulposus for expansion of the disc such that no portion of the disc annulus and the nucleus pulposus are removed. Also, what is needed is an apparatus and method for minimally invasive disc treatment which increases injectable volume at a lower pressure.

Accordingly, in one aspect, the invention provides an expandable device for intervertebral disc expansion comprising: a catheter; an inflatable member mounted on said catheter and insertable into a dilated opening in an intact intervertebral disc annulus and into a nucleus pulposus of the disc; and an inflation device connected to the inflatable member for controllable inflation of the inflatable member within the nucleus pulposus without removing any of the nucleus pulposus; said catheter has a first channel and a second channel whereby an inflation fluid for inflating said inflatable member can be evacuated via said first channel to deflate said inflatable member while a biomaterial is injected into the nucleus pulposus via the second channel.

In another embodiment, the invention provides a system including such a device, as defined by claim 14.

A principal advantage of this embodiment is that it enables disc expansion with a percutaneous or minimally invasive approach. The disc expansion enables a larger volume of biomaterial injection per treatment. A larger volume of biomaterial injection reduces the number of treatments to achieve desirable level of augmentation. This treatment enables disc expansion without removal of the nucleus pulposus and helps determine the appropriate biomaterial volume prior to injection. Over-injection of the disc, and resulting pain and complications, can be minimized using the proposed device and method. Another advantage is that the disc remains intact such that no portion of the disc annulus or disc nucleus is removed.

Preferred embodiments will now be described, by way of example only, with reference to the drawings.

Fig. 1 is a cross-sectional view illustrating an embodiment of a disc structure.

Figs. 2A-2F are cross-sectional views illustrating an embodiment of a disc expansion method and apparatus not falling under claim 1.

Fig. 3 is a cross-sectional view illustrating another embodiment of a disc expansion method and apparatus not falling under claim 1.

Fig. 4 is a cross-sectional view illustrating another embodiment of a disc expansion method and apparatus not falling under claim 1.

Figs. 5A-5D are cross-sectional views illustrating another embodiment of a disc expansion method and apparatus according to the invention.

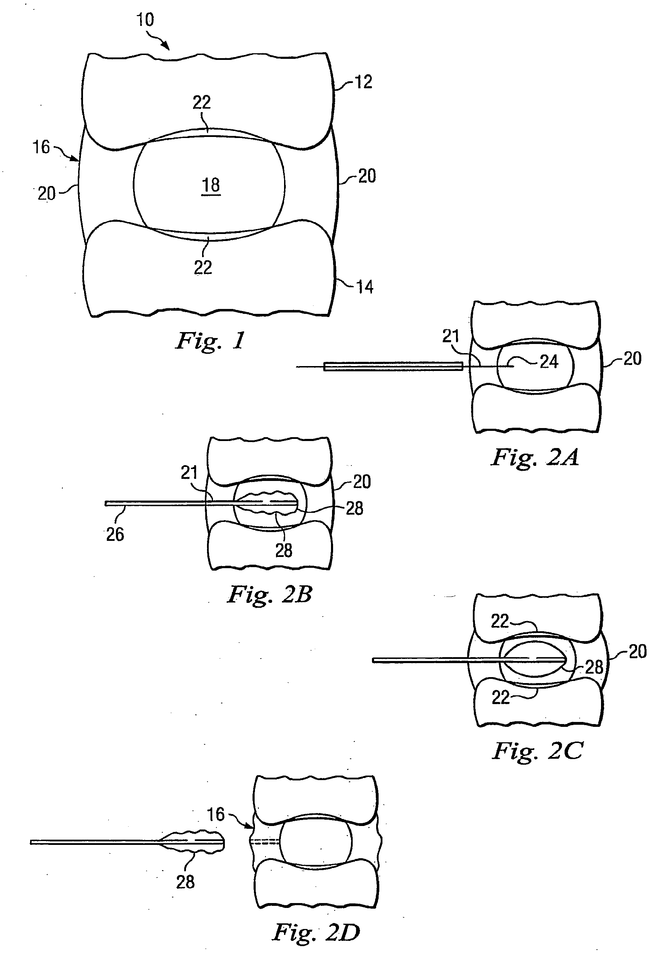

A disc structure 10, Fig. 1, generally comprises adjacent vertebrae 12 and 14 of the cervical, thoracic, or lumbar regions of the spine. An intervertebral disc 16 facilitates motion between the vertebrae 12 and 14 while absorbing shock and distributing loads. The disc 16 generally comprises a soft central core, i.e. the nucleus pulposus 18 (disc nucleus), that bears the majority of the load in a healthy disc, and a tough outer ring, i.e. the annulus fibrosis 20 (disc annulus), that surrounds and stabilizes the disc nucleus 18. A pair of cartilage endplates 22 are between each respective vertebrae 12 and 14, and the disc nucleus.
The method and apparatus are used following a patient diagnosis and selection for treatment, and in addition, a discogram to ensure disc annulus integrity.

The disc annulus 20, Fig. 2A, is punctured at 21 using a small diameter needle 24. A preferable needle size is 20 gauge. A small diameter (i.e. 1 to 3 mm) high-pressure balloon catheter 26, Fig. 2B, is introduced through the puncture 21 in the disc annulus 20. The location of a balloon 28 attached to catheter 26, in the disc nucleus 18 may be verified using fluoroscopy. The puncture required for insertion of devices for disc expansion and injection is small enough i.e. no greater than 3 mm, that the puncture may completely close, or close sufficiently that the injected biomaterial will remain captured. In the case of a biomaterial that sets up in the disc space after injection, capture of the injected biomaterial is assured. The use of an annulus closure device such as a plug or material such as a sealant is optional.

The balloon 28, Fig. 2C is gradually inflated with a saline and/or radiographic contrast medium such as sodium diatrizoate solution sold under the trademark Hypaque®, while monitoring the internal balloon pressure with a well known pressure gauge. Expansion of the balloon 28 is monitored using fluoroscopy. The rate of inflation and the pattern, size or shape of the balloon 28 can be varied between patients depending on disc condition. As the intradiscal pressure is increased and/or the endplates 22 are spread apart by the balloon 28, the disc annulus 20 is expected to stretch, as it is a viscoelastic material. The balloon may remain inflated from about 1 minute to about 1 hour, which may be varied for each patient. If significant expansion is required, the balloon may remain inflated up to 4 hours or it may be left in the disc space as a temporary implant up to 10 weeks.

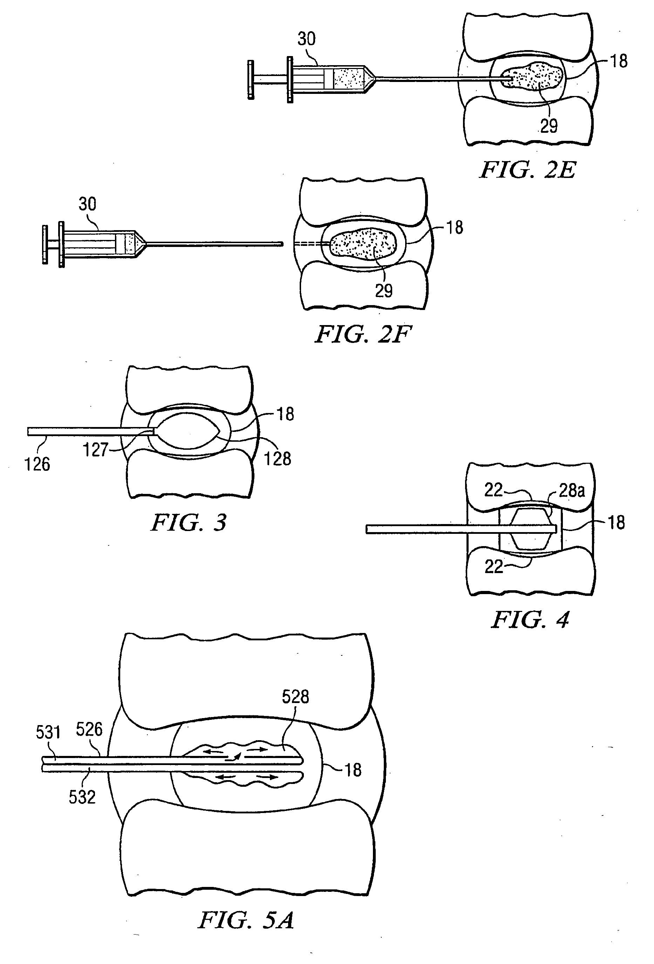

As the balloon 28, Fig. 2D, is deflated, the disc 16 becomes slack with an augmented space and reduced intradiscal pressure. Injectable biomaterial 29 such as a collagen gel can be delivered to the disc nucleus 18, Fig. 2E, through the same catheter. As the same catheter is used for injection, the injection can be done simultaneously as the balloon 28 is being deflated, as will be discussed below in greater detail.

Examples of biomaterials 29 which may be used for disc augmentation can be natural or synthetic, resorbable or non-resorbable. Natural materials include various forms of collagen that are derived from collagen-rich or connective tissues such as an intervertebral disc, fascia, ligament, tendon, skin, demineralized bone matrix, etc. Material sources include autograft, allograft, xenograft, human-recombinant origin, etc. Natural materials also include various forms of polysaccharides that are derived from animals or vegetation such as hyaluronic acid, chitosan, cellulose, agar, etc. Other natural materials include other proteins such as fibrin, albumin, silk, elastin and keratin. Synthetic materials include various implantable polymers or hydrogels such as silicone, polyurethane, silicone-polyurethane copolymers, polyolefin, polyester, polyacrylamide, polyacrylic acid, polyvinyl alcohol, polyethylene oxide, polyethylene glycol, polylactide, polyglycolide, poly(lactide-co-glycolide), poly(dioxanone), poly(ε-caprolactone), poly(hydroxylbutyrate), poly(hydroxylvalerate), tyrosine-based polycarbonate, polypropylene fumarate or combinations thereof. It is preferred that the biomaterial can undergo transition from a flowable to a non-flowable state shortly after injection. This can typically be achieved by adding a crosslinking agent to the biomaterial before, during, or after injection.

Proteoglycans may also be included in the injectable biomaterial 29 to attract and/or bind water to keep the disc nucleus 18 hydrated. Similarly, growth factors (e.g. transforming growth factor beta, bone morphogenetic proteins, fibroblast growth factors, platelet-derived growth factors, insulin-like growth factors, etc.) and/or other cells (e.g., intervertebral disc cells, stem cells, etc.) to promote healing, repair, regeneration and/or restoration of the disc, and/or to facilitate proper disc function, may also be included. Additives appropriate for use in the claimed invention are known to persons skilled in the art, and may be selected without undue experimentation.

Injectable biomaterial 29 is preferably mixed with the radiographic contrast medium prior to injection into the disc nucleus 18. This will allow the injection to be monitored using fluoroscopy. The catheter 26 or the needle 30, Fig. 2F, used for injection, is removed after an appropriate volume of biomaterial is deposited in the disc nucleus 18. As an alternative to withdrawing the balloon 28, as illustrated in Fig. 2D above, a balloon 128, Fig. 3 may be detachable at 127 from a catheter 126, and may remain inflated in the disc nucleus 18 as an implant. In the case of the detachable balloon 128, it may be advantageous to inject a biomaterial which, after injection, takes a set in an elastic or gel form. This could be accomplished by injecting a second material with the biomaterial which would alter the form of the injected material. In the case of direct injection of biomaterial 29 into the inflatable balloon member 28, the balloon 28 may be porous or permeable (e.g. woven fabric, mesh structure, perforated membrane, etc.) to allow material or fluid migration out of the inflatable member during or after injection.

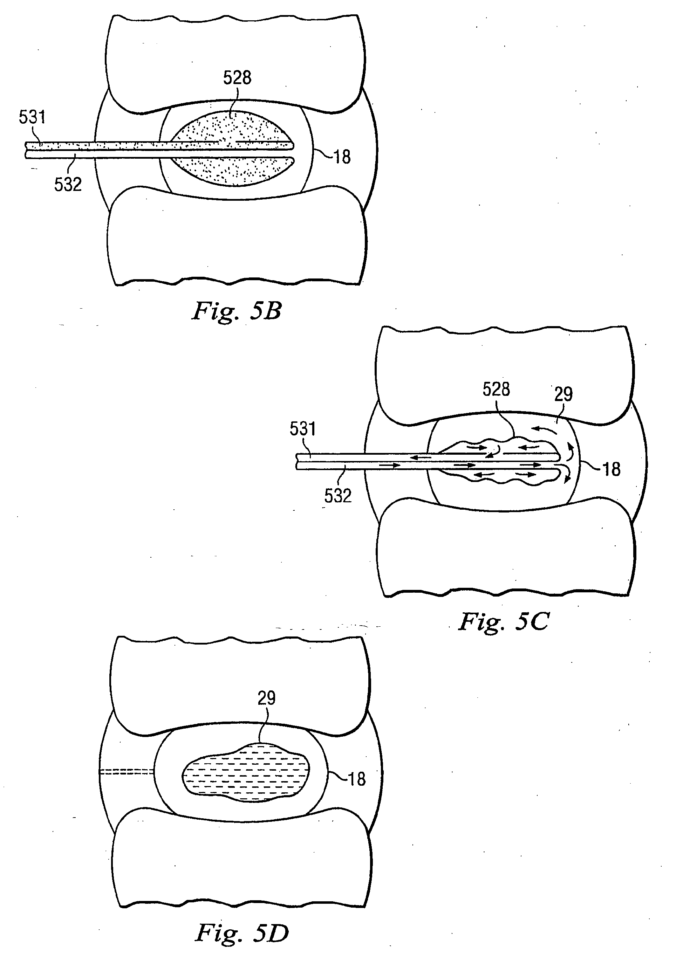

Alternatively, a modified balloon 28a, Fig. 4, may be of a shape including a profiler for inflating in a pattern for spreading the endplates 22 apart. That is, the balloon 28a is manufactured to expand to a suitable shape to better accomplish spreading the endplates 22 apart rather than to conform to the shape of disc nucleus 18 as in Fig. 2C. The balloon catheter according to the invention is a double lumen catheter which can be used for injection as the balloon is being deflated. In an embodiment, Fig. 5A illustrates a balloon catheter 526 introduced into the disc nucleus 18. The catheter 526 includes a first channel 531, a second channel 532 and a balloon 528. The saline and/or radiographic contrast medium is injected into balloon 528 via the first channel 531 to inflate balloon for expansion of the disc nucleus 18. In Fig. 5B, the inflated balloon 528 remains inflated in the disc nucleus 18 for an appropriate amount of time to stretch the annulus fibrosis and/or expand the nuclear disc space. In Fig. 5C, an appropriate biomaterial 29 is injected into the disc nucleus 18 via the second channel 532 in catheter 526 while the balloon inflating medium is simultaneously evacuated via the first channel 531. In Fig. 5D, the deflated balloon 528 is withdrawn with catheter 526 from the disc nucleus 18 and the injected biomaterial 29 remains within the disc nucleus 18.

As a result, one embodiment provides an apparatus including a high-pressure balloon catheter with a small shaft diameter (3mm or smaller, preferably 2 mm or smaller, most preferably 1mm or smaller). The catheter has a pointed tip for puncturing an intact disc annulus and insertion of the balloon section into the nuclear disc region. The catheter either has rigid shaft or is supported by a rigid guide-needle during penetration into the disc. For a rigid shaft, the catheter can be made of metal tubing. For a flexible shaft, the catheter can be made of polymeric tubing and is supported with a rigid guide-needle or guide-wire. If a guide-needle is used, the catheter can be double lumen. The balloon has an appropriate final volume of from about 0.1 cc to about 8.0 cc, preferably up to 5.0 cc and dimensions (length = 5-40 mm, preferably 10-30 mm; diameter = 3-20 mm, preferably 5-15 mm) to fit the nuclear disc region. The balloon can be of various shapes; conical, spherical, square, long conical, long spherical, long square, tapered, stepped, dog bone, offset, or combinations thereof. Balloons can be made of various polymeric materials such as polyethylene terephthalates, polyolefins, polyurethanes, nylon, polyvinyl chloride, silicone, polyetheretherketone, polylactide, polyglycolide, poly(lactide-co-glycolide), poly(dioxanone), poly(ε-caprolactone), poly(hydroxylbutyrate), poly(hydroxylvalerate), tyrosine-based polycarbonate, polypropylene fumarate or combinations thereof.

A comparative example provides first, a determination that the treated disc has a competent and intact annulus fibrosis for safe expansion and effective containment of the subsequently injected biomaterial. After the annulus quality and integrity are verified using discography, the disc expansion device with the smallest shaft diameter possible, is inserted into the center of the disc. Insertion of the device can be done percutaneously, preferably under fluoroscopic guidance. The balloon is gradually inflated with radio-contrast fluid or saline to pressurize the disc, and thereby, stretch the annulus fibrosis. After a predetermined inflation time, the balloon is deflated and removed from the disc space.
Since a double-lumen catheter is employed, the biomaterial can be injected into the disc through the same catheter during or after balloon deflation. The whole procedure is preferably done under fluoroscopic guidance.

The foregoing has described an apparatus for expansion of an intervertebral disc prior to its augmentation with an injectable biomaterial. Disc expansion prepares the disc annulus to receive a desirable or effective volume of injectable material in a single treatment. Because the annulus fibrosis is a viscoelastic material, it can be temporarily stretched as the disc is expanded under pressure.

## Claims

1. An expandable device for intervertebral disc expansion comprising:
a catheter (526);
an inflatable member (528) mounted on said catheter and insertable into a dilated opening in an intact intervertebral disc annulus and into a nucleus pulposus of the disc; and
an inflation device (526) connected to the inflatable member for controllable inflation of the inflatable member within the nucleus pulposus without removing any of the nucleus pulposus; said catheter having a first channel (531) and a second channel (532) **characterized in that** an inflation fluid for inflating said inflatable member can be evacuated via said first channel to deflate said inflatable member while a biomaterial is injected into the nucleus pulposus via the second channel.

2. The expandable device as defined in claim 1 wherein the inflatable member has a controlled expanded shape conforming substantially to the nucleus pulposus shape.

3. The expandable device as defined in claim 2 wherein the inflatable member has an inflated volume of from 0.1 cc to 8.0 cc.

4. The expandable device as defined in any preceding claim, further comprising:
a profiler formed in the inflatable member and shaped for spreading vertebral endplates apart in response to expansion of the inflatable member.

5. The device as defined in claim 1 wherein the biomaterial is provided as a formulation that includes growth factors.

6. The device as defined in claim 1 wherein the biomaterial is provided as a formulation that includes one or more types of cells effective to promote healing, repair, regeneration and/or restoration of the disc, and/or facilitate proper disc function.

7. The device as defined in claim 1 wherein the biomaterial is altered from a flowable state to a non-flowable state after inflation of the inflatable member.

8. The device as defined in claim 7 wherein the biomaterial is a synthetic material.

9. The device as defined in claim 8 wherein the synthetic material is a polymer.

10. The device as defined in claim 8 wherein the synthetic material is a hydrogel.

11. The device as defined in claim 1 wherein the biomaterial is a natural material.

12. The device as defined in claim 11 wherein the natural material is a collagen material.

13. The device as defined in claim 11 wherein the natural material is a polysaccharide material.

14. An expansion and injection system for an intervertebral disc comprising an expandable device as claimed in any preceding claim, and further comprising:
an instrument for forming and dilating an opening in the disc annulus without removing any of the disc annulus;
a gauge for monitoring the inflatable member pressure;
means for deflating the inflatable member; and
an injection instrument (30) for injecting the biomaterial into the augmented space.

15. The system as defined in claim 14 wherein the inflatable member (28) is adapted such that its location is verified by fluoroscopy.

16. The system as defined in claim 14 wherein the inflatable member (28) is adapted to be inflated with a radio contrast material.

17. The system as defined in claim 14, wherein the inflatable member is adapted such that its expansion is monitored by fluoroscopy.

## Patentansprüche

1. Erweiterbare Vorrichtung zur Bandscheibenerweiterung, umfassend
einen Katheter (526);
ein aufblasbares Element (528), das an dem Katheder befestigt ist und in eine geweitete Öffnung in einem intakten Bandscheibenring und in einen Nukleus Pulposus in der Scheibe einsetzbar ist; und
eine Aufblasvorrichtung (526), die mit dem aufblasbaren Element zum gesteuerten Aufblasen des aufblasbaren Elements in dem Nukleus Pulposus ohne Entfernen von jeglichem Nukleus Pulposus verbunden ist, wobei der Katheter einen ersten Kanal (531) und einen zweiten Kanal (532) aufweist, **dadurch gekennzeichnet, dass** ein Aufblasfluid zum Aufblasen des aufblasbaren Elements über den ersten Kanal entleert werden kann, um das aufblasbare Element zu entleeren, während über den zweiten Kanal ein Biomaterial in den Nukleus Pulposus injiziert wird.

2. Erweiterbare Vorrichtung wie in Anspruch 1 definiert, wobei das aufblasbare Element eine gesteuerte erweiterte Gestalt aufweist, die im Wesentlichen der Gestalt des Nukleus Pulposus entspricht.

3. Erweiterbare Vorrichtung wie in Anspruch 2 definiert, wobei das aufblasbare Element ein Aufblasvolumen von 0,1 cc bis 8,0 cc aufweist.

4. Erweiterbare Vorrichtung wie in einem der vorangehenden Ansprüche definiert, ferner umfassend:
eine Profilkorrektureinrichtung, die in dem aufblasbaren Element ausgebildet und derart gestaltet ist, dass sie als Antwort auf die Erweiterung des aufblasbaren Elements Endplatten der Wirbelsäule aufspreizt.

5. Vorrichtung wie in Anspruch 1 definiert, wobei das Biomaterial als eine Formulierung bereitgestellt ist, die Wachstumsfaktoren einschließt.

6. Vorrichtung wie in Anspruch 1 definiert, wobei das Biomaterial als eine Formulierung bereitgestellt ist, die einen oder mehrere Zelltypen einschließt, der/die zum Fördern der Heilung, Wiederherstellung, Regeneration und/oder Erneuerung der Scheibe und/oder zum Ermöglichen einer ordnungsgemäßen Scheibenfunktion wirksam ist/sind.

7. Vorrichtung wie in Anspruch 1 definiert, wobei das Biomaterial nach dem Aufblasen des aufblasbaren Elements von einem fließfähigen Zustand zu einem nichtfließfähigen Zustand umgewandelt wird.

8. Vorrichtung wie in Anspruch 7 definiert, wobei es sich bei dem Biomaterial um ein synthetisches Material handelt.

9. Vorrichtung wie in Anspruch 8 definiert, wobei es sich bei dem synthetischen Material um ein Polymer handelt.

10. Vorrichtung wie in Anspruch 8 definiert, wobei es sich bei dem synthetischen Material um ein Hydrogel handelt.

11. Vorrichtung wie in Anspruch 1 definiert, wobei es sich bei dem Biomaterial um ein natürliches Material handelt.

12. Vorrichtung wie in Anspruch 11 definiert, wobei es sich bei dem natürlichen Material um ein Kollagenmaterial handelt.

13. Vorrichtung wie in Anspruch 11 definiert, wobei es sich bei dem natürlichen Material um ein Polysaccharidmaterial handelt.

14. Erweiterungs- und Injektionssystem für eine Bandscheibe, umfassend eine erweiterbare Vorrichtung wie in einem der vorangehenden Ansprüche definiert und ferner umfassend:
ein Instrument zum Bilden und Weiten einer Öffnung in dem Scheibenring ohne Entfernen von jeglichem Scheibenring;
eine Messeinrichtung zum Überwachen des Drucks des aufblasbaren Elements;
Mittel zum Entleeren des aufblasbaren Elements; und
ein Injektionsinstrument (30) zum Injizieren des Biomaterials in den erweiterten Raum.

15. System nach Anspruch 14, wobei das aufblasbare Element (28) derart angepasst ist, dass seine Lokalisierung durch Fluoroskopie nachgewiesen werden kann.

16. System wie in Anspruch 14 definiert, wobei das aufblasbare Element (28) derart angepasst ist, dass es mit einem Radiokontrastmaterial aufgeblasen werden kann.

17. System wie in Anspruch 14 definiert, wobei das aufblasbare Element derart angepasst ist, dass seine Erweiterung durch Fluoroskopie überwacht werden kann.

## Revendications

1. Dispositif expansible pour une expansion de disque intervertébral comprenant :
- un cathéter (526) ;
- un élément gonflable (528) monté sur ledit cathéter et apte à être introduit dans une ouverture dilatée dans un annulus de disque intervertébral intact et dans un noyau gélatineux du disque ; et
- un dispositif de gonflage (526) relié à l'élément gonflable pour un gonflage commandable de l'élément gonflable à l'intérieur du noyau gélatineux sans retirer une quelconque partie du noyau gélatineux, ledit cathéter ayant un premier canal (531) et un second canal (532), **caractérisé par le fait qu'**un fluide de gonflage pour gonfler ledit élément gonflable peut être évacué par l'intermédiaire dudit premier canal pour dégonfler ledit élément gonflable tandis qu'un biomatériau est injecté dans le noyau gélatineux par l'intermédiaire du second canal.

2. Dispositif expansible selon la revendication 1, dans lequel l'élément gonflable a une forme expansée commandée se conformant sensiblement à la forme du noyau gélatineux.

3. Dispositif expansible selon la revendication 2, dans lequel l'élément gonflable a un volume gonflé de 0,1 cm³ à 8, 0 cm³.

4. Dispositif expansible selon l'une quelconque des revendications précédentes, comprenant en outre :
- un organe de profilage formé dans l'élément gonflable et façonné pour étaler les plaques d'extrémité vertébrales en réponse à une expansion de l'élément gonflable.

5. Dispositif selon la revendication 1, dans lequel le biomatériau est fourni sous forme d'une formulation qui comprend des facteurs de croissance.

6. Dispositif selon la revendication 1, dans lequel le biomatériau est fourni sous la forme d'une formulation qui comprend un ou plusieurs types de cellules efficaces pour favoriser une cicatrisation, une réparation, une régénération et/ou une restauration du disque, et/ou faciliter une fonction de disque correcte.

7. Dispositif selon la revendication 1, dans lequel le biomatériau est modifié à partir d'un état apte à s'écouler à un état non-apte à s'écouler après gonflage de l'élément gonflable.

8. Dispositif selon la revendication 7, dans lequel le biomatériau est un matériau synthétique.

9. Dispositif selon la revendication 8, dans lequel le matériau synthétique est un polymère.

10. Dispositif selon la revendication 8, dans lequel le matériau synthétique est un hydrogel.

11. Dispositif selon la revendication 1, dans lequel le biomatériau est un matériau naturel.

12. Dispositif selon la revendication 11, dans lequel le matériau naturel est un matériau de collagène.

13. Dispositif selon la revendication 11, dans lequel le matériau naturel est un matériau de polysaccharide.

14. Système d'expansion et d'injection pour un disque intervertébral comprenant un dispositif expansible tel que défini à l'une quelconque des revendications précédentes, et comprenant en outre :
- un instrument pour former et dilater une ouverture dans l'annulus de disque sans retirer une quelconque partie de l'annulus de disque ;
- une jauge pour surveiller la pression de l'élément gonflable ;
- des moyens pour dégonfler l'élément gonflable ; et
- un instrument d'injection (30) pour injecter le biomatériau dans l'espace augmenté.

15. Système selon la revendication 14, dans lequel l'élément gonflable (28) est adapté de telle sorte que son emplacement est vérifié par fluoroscopie.

16. Système selon la revendication 14, dans lequel l'élément gonflable (28) est adapté pour être gonflé avec un matériau de contraste radio.

17. Système selon la revendication 14, dans lequel l'élément gonflable est adapté de telle sorte que son expansion est surveillée par fluoroscopie.
